# FASCICULE DE BREVET EUROPEEN

(11) **EP 0 969 851 B1**
(45) Date de publication et mention de la délivrance du brevet: **26.05.2004**
(21) Numéro de dépôt: 97906249.4
(22) Date de dépôt: 25.02.1997
(51) Int. Cl.: A61K 35/74, A61K 39/106

(54) **COMPLEXE IMMUNOMODULATEUR ET SON UTILISATION DANS LES AFFECTIONS PAR HELICOBACTER**
IMMUNMODULATORISCHER KOMPLEX UND DESSEN VERWENDUNG IN HELICOBACTER ERKRANKUNGEN
IMMUNOMODULATORY COMPLEX AND USE THEREOF IN HELICOBACTER DISEASES

(30) Priorité: 26.02.1996 FR 9602445
(43) Date de publication de la demande: 12.01.2000
(73) Titulaire: TOROSSIAN, Fernand Narbey, F-31400 Toulouse (FR)
(72) Inventeur: TOROSSIAN, Fernand Narbey, F-31400 Toulouse (FR)
(74) Mandataire: Morelle, Guy Georges Alain
(86) Numéro de dépôt international: PCT/FR1997/000334
(87) Numéro de publication internationale: WO 1997/030716

(56) Documents cités:
- EP-A- 0 035 429
- WO-A-94/22462
- CLINICAL IMMUNOTHERAPEUTICS, vol. 4, no. 2, 1995, pages 138-146, XP000609056 FAURE G. ET AL.: "Use of Bacterial Ribosomal Immunostimulators in Respiratory Tract Infections"

## Description

La présente invention concerne un complexe vaccinal thérapeutique et préventif anti-bactérien. qui possède un pouvoir vaccinant lié à la présence d'antigènes spécifiques contre l'Helicobacter pylori (antérieurement appelé Campylobacter pylori) l'Helicobacter hepaticus. l'Helicobacter coronari, et non spécifiques assurant une immunomodulation.

[MARSHALL BJ. WARREN Jr.. Unidentified curved bacilli in the sto-mach of patients with gastritis and peptic ulceration *Lancet* 1984: i:1311-4)]

[MÉGRAUD F. *Helicobacter pylori,* chef de file des bactéries du mucus. La lettre de *l'infectiologue* 1993: 8 (suppl. 4): 151-9].

Il est bien connu, en bactériologie. que les antigènes de surface des parois, des membranes ou des capsules (combinés ou libérés sous forme soluble dans le milieu de culture) sont de nature glycoprotéique, polypeptidique ou polysaccharidique.

Des vaccins associant à l'acide ribonucléique d'origine ribosomale (ARN) des facteurs associatifs, telles que des substances membranaires protéoglycaniques ou polysaccharidiques. extraites de germes pathogènes. sont utilisables dans l'élaboration des vaccins acellulaires (cf. Inf. and Immunity, 1, 574-82. 1970 et PCT WO 94/22462).

Ces vaccins utilisent des antigènes spécifiques correspondant à des affections microbiennes spécifiquement déterminées.

Or , le pouvoir antigénique est essentiellement lié au niveau de l'ARN (des ribosomes en particulier) des cellules microbiennes, entre autres. Les Cellules Immunologiquement Compétentes (CIC) utilisent directement ces ARN comme transporteurs actifs.

Pour élaborer le complexe de l'invention, avec l'antigène sérotype bactérien d'Helicobacter, nous avons couplé, gràce à des liaisons de préférence covalentes, l'ARN d'origine ribosomale, de préférence, à une séquence d'acides aminés de nature glycoprotidique, présente de préférence dans le collagène de type III. Chez l'homme, le collagène représente approximativement le tiers des protéines de l'organisme. Le type III a été choisi pour sa séquence d'acides aminés et sa présence dans le derme, la paroi vasculaire et les muqueuses épithéliales digestives.

Dans notre complexe, nous utilisons comme stabilisant des fractions membranaires cellulaires issues des mêmes germes que ceux qui ont servi à l'élaboration de l'ARN ribosomal. Ces fractions membranaires contiennent la totalité des substances peptidoglycaniques et sont connues en outre comme adjuvants d'immunité.

Il est, en plus d'Helicobacter pylori, hepaticus, et coronari, utile d'avoir des fractions membranaires - glucopolysacchariques ou protéogly-canes - issues de différents germes microbiens qui ont servi à fournir l'ARN par extraction de leurs ribosomes, germes connus pour leur immunogénèse (recrutement de macrophages, activation de lymphocytes T, potentialisation de la synthèse des immunoglobulines. IgA sécrétoires notamment (11 S), augmentation de la phagocytose et stimulation des cellules T dépendantes...).

Ceci a été ainsi conçu car, dans le cas précis des pathogénèses induites par Helicobacter pylori, hepaticus ou helmannii, coronari, l'organisme doit élaborer en plus de la réponse immunitaire spécifique humorale, une réponse cellulaire pour pallier l'inefficacité des anticorps dans la protection de l'individu.

Il est connu que la réponse à la médiation cellulaire ne donne pas lieu à la production d'anticorps, mais seulement à la génération des cellules lymphoïdes sensibilisées et spécifiques de l'antigène en cause.

Les lymphocytes T agissent par eux-mêmes et/ou par les cytokines, et on observe soit une réponse de type inflammatoire, soit une réponse cyto-toxique.

Le pouvoir pathogène d'Helicobacter réside dans son aptitude à coloniser la muqueuse gastrique, à survivre dans le suc gastrique, et à s'y multiplier en dépit de la réponse immunitaire de l'hôte, et à générer des lésions parfois irréversibles (adénocarcinome, lymphome gastrique ou lymphomes de MALT "mucuous associated lymphoid tissue"),

[PARSONNET J: Helicobacter pylori and gastric cancer, Gastroenterol Clin North Am 1993. 22:89-104.

WORTHERSPOON AC, DOGLIONI C, DISS TC et al: Régression of primary low-grade B-cell gastric lymphoma of mucosa associated lymphoid tissue type after eradication of Helicobacter pylori, Lancet 1993: 342:575-7.

MOHANDAS, Helicobacter pylori and lymphoma, N Eng J Med 1994: 331:746-7]. lorsque celle-ci est insuffisante lors de l'injection : résistance à la phagocytose, induction d'apoptose..,etc.

[PETERSON, P.K., VERHOEF. J., SCHMELING, D. & QUIE. P.G.: Kinetics of phagocytocis and bacterial killing by human polymorphonuclear leucocytes and monocytes, J. Infec. Dis. 136:502-509, 1977.

KIEHLBAUCH JA, ALBACH RA, BAUM I.L, CHANG KP, Phagocytosis of Campylobacter jejuni and its intracellular survival in mononuclear phagocytes, Infect Immun 1985: 48:446-51].

### Constituants du complexe vaccinal objet de l'invention

Le complexe de l'invention comprend des molécules duales constituées par le couplage d'un bras fonctionnel d'acides aminés assurant la liaison à une cible, avec un bras génétique d'ARN correspondant à la description codée de la composition du bras fonctionnel.
A - Les ARN d'origine ribosomale utilisables peuvent être extraits des souches choisies dans le groupe suivant, cette liste n'étant pas limitative :
   - Helicobacter pylori (ou Campylobacter), hepaticus, coronari ...
   - Klebsiella pneumoniae
   - Streptococcus (pneumoniae et pyogènes)
   - Staphilococcus aureus
   - Serratia marcescens .
   - Escherichia coli :
   - Salmonella typhimurium
   - Corynebacterium (granulosum, parvum. acnes)
   - Mycobacterium (tuberculosis. smegmatis. chelonei)
   - Hemophilus influenzae
   - Pneumocoque type Il
   - Rothia dento cariosus
   - Bacterium coli
   - Shigella dysentariae
   - Enterococcus
   - Nocardia (astéroïdes, brasiliensis, rhodocrans, opaca, rubra)
   - Bacille de Calmette et Guerin.
   ou d'un mélange de celles-ci.
   Les poids moléculaires moyens de ces ARN se situent entre 5 104 et 108 Dalton.
   De multiples procédés industriels existent pour la préparation de l'ARN. Nous citerons comme exemple le procédé d'extraction d'ARN décrit dans Infect, and Immunity, 1, 574-82. 1970 : les bactéries sont broyées puis soumises à une précipitation fractionnée, les protéines ribosomales sont solubilisées, l'ARN précipité est traité par Pronase et, enfin, purifié par chromatographie échangeuse d'ions.
   Si l'ARN est obtenu par voie enzymatique, la purification finale peut être faite par chromatographie de tamisage moléculaire. Voir notamment à ce sujet :
   - C. EHRESMAN (1972) - Biochimie, 54, 901
   - H. KAGAWA (1972) - J. Biochem., (1972), 827
   - M. SANTER (1973) - J. Bact., 116, 1304
   - NOMURA (1974) - Ribosomes - Ed. Cold Spring Harbor Laboratory.
B - Les fractions membranaires de cellules bactériennes utilisables peuvent être extraites des souches suivantes, les listes données n'étant pas limitatives :
   1- pour les polysaccharides capsulaires
      a. Helicobacter pylori et hepaticus
      b. Klebsiella pneumoniae
      c. Streptococcus pneumoniae
      d. Hemophilus influenzae
      e. Escherichia coli
         a. Helicobacter pylori, hepaticus, et coronari
            [HILLS BA, Gastric mucosal barrier: evidence for Helicobacter pylori ingesting gastric surfactant and deriving protection from it, Gut, 1993 May: 34(5):588-93.
            GENTA RM; ROBASON GO; GRAHAM DY, Simultaneous visualization of Helicobacter pylori and gastric morphology: a new stain, Human Pathology: 1994 Mar: 25(3):221-6.
            MAJEWSKI. S.I., and C.S GOODWIN, 1988, Restriction endonuclease analysis of the genome of Campylobacter pylori with a rapid extraction method: evidence for considerable genomic variation, J. Infect. Dis. 157:465-471.
            GEIS. G., LEYING. H., SUERBAUM. S., MAI. U. & OPFERKUCH. W.: Ultrastructure and chemical analysis of Campylobacter pylori flagella, J. Clin. Microbiol. 27:436-441, 1989].
         b. Klebsiella pneumoniae
            [- C. ERBING, L. KENNE, B. LINBERG, J. LONNGREN (1976) - Structural studies of the capsular polysaccharide of Klebsiella pneumoniae type I (Carbohydr. Res., 50 (1976) 115-20).
            - W. NIMMICH (1968) - Zur Isolierung und qualitativen Bausteianalyse der K. Angigen von Klebsiellen (Med. Mikrobio. und Immunol., 154, 117, 131).
            - C. RICHARD (1973) - Etude antigénique et biochimique de 500 souches de Klebsiella
               (Ann. Biol. Clin., 1973)].
         c. Streptococcus pneumoniae :
            [- F. KAUFFMANN et E. LUND (1954) (Int. Bull. Bact. Nomencl. 4, 125-28).
            - FELTON et OTTINGER (J. of Bacteriology, 1942, 43, 94, 105)
            - M. COLIN. M.D. MAC LEOD et coll. - Prévention of pneumococcal pneumoniae by immunization with specific capsular polysaccharides (J. Exp. Med., 1945, 82, 445-65).
            - A.R. DOCHEZ et O.T. AVERY - The elaboration of specific soluble substance by Pneumococcus during growth (1971) (J. Exp. Med. 26, 477-93).
            - WEST PHAL et LUDERITZ (1952) (Z. Naturf. 7B, 148).
            - C.P.J. GLAUDEMANS et H.P. TREFFERS - An improved préparation of the capsular polysaccharide from from Diplococcus pneumoniae (Carbon dr. Res. 1967, 4, 181-84)].
         d. Hemophilus influenzae (polysaccharide capsulaire de type poly-ribose-phosphate)
            [- P. ANDERSON et coll. (1972) - Immunisation of humans with polyribosephosphate, the capsular antigen of Hemophilus influenzae type B (J. of Clin. Invest., vol. 51, 1972, 39-44).
            - P. ANDERSON et coll. (1977) - Isolation of the capsular polysaccharide from supernatant of Hemophilus influenzae type B (Infect. and Immun., 1977, 15 (2), 472-77)]
         e. Escherichia coli (polysaccharides capsulaires)
            [- LUDERITZ et coll. (1977) - Somatic and capsular antigens of gram-negative bacteria (Compr. Biochem. 26 A, 105-228).
            - BOYER H.W., and D. ROULLAND-DISSOIX, (1969) - A complementation analysis of the restriction and modification of DNA in Escherichia *coli.* J. Mol. Biol. (41:459-472).
            - CASADABAN. M., and S. N. COHEN ( 1980) - Analysis of gene control signals by DNA fusion and cloning in E. coli, J. Mol. Biol. (138:179-207).
            - LUGTENBERG. B., J. Meijers, R. Peters, P. van der Hock, and L. van Alphen (1975) - Electrophoretic résolution of the "major outer membrane protein" of Escherichia coli K12 into four bands, (FEBS Lett. 58:254-258)1.
   2 Pour les lipopolysaccharides membranaires (LPS)- Corynebacterium (avidum, bovis, diphteriae, enzymicum, equi, fascians, flaccum, faciens, flavidum, fusiforme, granulosum, helvolum, hypertrophicans, insidiosum, liquefaciens, parvum, paurometabolum, pyogènes, tumescens, xerosis)
      - et les gram-moins :
      - Helicobacter pylori, hepaticus, coronari
      - Klebsiella (pneumoniae et rhinoscleromatis)
      - Salmonella typhimurium
      - Serratia (marcescens, corralina, indica, plymuthica, kituea)
      - Neisseria meningitidis
      - Escherichia coli
         [GOODWIN C. S. "Helicobacter Pylori : 10th anniversary of its culture in April 1982", (Gut 1993 ; 34 : 293-4).
      - C. ERBIN et coll. (1977) - Structural studies on the Klebsiella LPS (Carbohydr. Res., 56. 377-81).
      - C.B. CASTOR et coll. (1971) - Characteristics of a highly purified pyrogenic LPS of Klebsiella pneumoniae (J. of Pharm. Sci., 60, (10), 1578-80).
      - K. FUKUSHI ( 1964) - Extraction and purification of endotoxin from Enterobacteriaceae: a comparison of selected methods and sources (J. of Bacteriol. 87, (2)n 391-400).
      - G.A. LIMJUCO - Studies on the chemical composition of LPS from Neisseria meningitidis group B (J. of Gen. Microbiol. 1978, 104, 187-91).
      - G.A. ADAMS ( 1967) - Extraction of LPS from gram-negative bacteria with DMSO (Canad. J. Biochem., 45, 422-26).
      - K.G. JOHNSON (1976) - Improved techniques for the préparation of bacterial LPS (Canad. J. Microbiol. (22), 29-34).
      - Y.B. KIM et coll. (1967) - Biologically active endotoxins from Salmonella mutans (J. of Bacteriol., 94, (5), 1320-26)].
   3 - Pour les protéines membranaires
      - Helicobacter Pylori
      - Escherichia coli
      - Serratia marcescens
      - Streptococcus pyogènes
      - Salmonella typhimurium.
         Helicobacter Pylori, Hepaticus, Coronari ...
      - GOBERT (B.), LABIGNE (A.), de KORWIN (J.D.), CONROY (M.C.), BENE (M.C.), FAURE (G.C.) - Polymerase chain reaction for Helicobacter pyroli, (Rev. Esp. Enf Digest. 1980, 78 (suppl 1), 4.
      - TOWBIN. H., T. STAEHELIN, and J. GORDON, 1979, Electrophoretic transfer of proteins from polyacrylamide gels to nitrocellulose sheets: procedure and some applications, Proc. Natl. Acad. Sci. USA 76:4350-4354,
         Escherichia coli
      - S.F. STIRM et coll. ( 1967) - Episome. carried surface antigen K 88 of Escherichia coli (J. of Bacteriol., 93, (2), 731-39),
      - S.J. BETZ et coll. (1977) - Chemical and biological properties of a protein rich fraction of bacterial LPS (J. of Immunol., 119, (4), 1475-81),
         Serratia marcescens
      - W. WOBER (1971) - Studies on the protein moiety of endotoxin from gram-negative bacteria. characterisation of the protein-moieting isolated by acetic acid hydrolysis of endotoxin of Serratia marcescens.
         Streptococcus pyogènes
      - M.K. WITTNER ( 1977) - Homologous. and heterologous protection of mice with group-A Streptococcal M protein vaccine (Infect. and Immun., 1977, 15, (1), 104-8).
         Salmonella thyphimurium
      - N. KUUSI et coll. (1979) - Immunization with major outer membrane protein in expérimental salmonellosis of mice (Infect. and Immun., 1979, 25, (3), 857-62).
      - C. BARBER et coll. ( 1972) - The protective rôle of proteins from Salmonella thyphimurium in infection of mice with their natural pathogen (Rev. Immunol., 36, 77-81).
      - G. DELORD ( 1979) - Etude d'un antigène vaccinant contenu dans le surnageant de culture de Salmonella thyphimurium. souche M-206. thèse de médecine de Lyon n° 428, 1979.
      - G.W. GOODMAN (1979) - Characterization of the chemical and physical properties of a novel B-lymphocyte activator endotoxin protein (Infect. and Immun., 1979, 24 (3), 685-96).
   4 - Pour les acides teichoïques et lipoteichoïques
      Streptocoques, staphylocoques, et lactobacilles (la surface des bactéries gram-positives est faite d'acide teichoïque, qui est un polymère du glycérol, lié par des ponts phosphodiesters).
      Les articles suivants décrivent les procédés d'obtention :
      - M.M. BURGER (1966) - Teichoïc acids: antigenic déterminants, chain séparation, and their location in the cell wall (Microbiology 56, 910-17).
      - K.W. KNOX (1973) - Immunological properties of teichoïc acids (Bacteriol. Reviews, 37, 21, 215-57).
      - G.A. MILLER (1976) - Effects of streptococcal lipoteichoïc acid on host response in mice (Infect. and Immun., 1976, 13, (5), 1408-17).
      - A.J. WICKEN et coll. (1975) - Lipoteichoïc acids: a new class of bacterial antigens (Science, 187, 1161-67).
      **Différents dosages possibles**
      A.R.N.
         * FISKE et SUBBAROW - Dosage du phosphore. Chromatographie HPLC sur colonne échangeuse d'ions pour le contrôle qualitatif (J. Biol. Chem. (1926), 66, 375).
      Protéines
         * LOWRY (J. Biol. Chem. (1951), 193, 265-75).
      Hexoses
         * T.A. SCOTT - Dosage colorimétr. à l'anthrone (Anal. Chem. (1953), 25, 1956-61).
      Hexosamines
         * L.A. ELSON (Biochem. J (1953), 27, 1824-28).
      Lipopolysaccharides
         * J. JANDA et E. WORK (Febs Letters, 1971, 16 (4), 343-45).
C - Les autres facteurs adjuvants de l'immunité, en plus des fractions membranaires, sont
   - du collagène type III
   - du chlorure de sodium
   Le collagène de type III utilisé est caractérisé par :
   a - des séquences d'acides aminés voisines de la séquence suivante (les concentrations sont exprimées en g/kg) :
      - acide aspartique AA 51.5
      - hydroxyproline HP 107.0
      - thréonine TH 16.1
      - sérine SE 27.8
      - acide glutamique AG 95,9
      - proline PR 124,0
      - glycine GL 149,0
      - alanine AL 87,9
      - valine VA 23,3
      - méthionine ME 7,5
      - isoleucine IL 14,4
      - leucine LE 27,8
      - tyrosine TY 6,7
      - phénylalanine PA 14,4
      - lysine LY 28,6
      - histidine HI 5,5
      - arginine AR 73,0
   b - l'analyse-type suivante :
      - couleur blanc jaunâtre
      - densité apparente 250 g/l
      - humidité 6 %
      - pH d'une solution à 10 % 6,9
      - viscosité Engler à 40°C 2,5
   (solution à 17,75 %)
   - taux de matières grasses 0,9 %
   - taux de cendres 2,2 %
   - taux de Fe + Cu + Ca 462 mg/kg
   - métaux lourds non décelables par spectrographie d'émission d'arc
   - analyse élémentaire C 46,80 %
   H 7,10%
   N 14,96 %

La composition du complexe vaccinal objet de l'invention, associant des ARN ou des fragments d'ARN ribosomaux. des fractions membranaires (par exemple protéoglycanes de Klebsiella pneumoniae) et du collagène de type III, complété par du chlorure de sodium et un anti-inflammatoire, permet, par administration de faibles doses n'entraînant aucune toxicité, d'obtenir un haut niveau de protection et de guérison.

La présentation préférée est la forme injectable de la composition ci-dessus présentée, mais il est possible d'utiliser d'autres présentations et/ou d'autres supports ou additifs compatibles avec une utilisation médicale.

### Mécanisme d'action du complexe vaccinal

Ce complexe thérapeutique (vaccinal) peut être assimilé à un vaccin spécifique (par "système inerte" qui a pour but d'augmenter l'immunogénicité d'un vaccin recombinant sous-unité et des vaccins constitués des peptides), et d'un vaccin non spécifique avec les caractéristiques d'une lymphokine, qui, se fixant aux macrophages, joue un rôle essentiel dans la réponse immune vis-à-vis d'Helicobacter [KAZI, J.L, SINNIAH, R. JAFFRAY, N.A., ALAM, S.M., ZAMAN. V., ZUBERI. S.J. & KAZI. A.M.: Cellular and humoral immune responses in Campylobacter pylori-associated chronic gastritis, J. Pathol. 159: 231-237, 1989].

Depuis 1974-75 (A.S.et G.P. YOUMANS ), il a été constaté que l'effet d'inhibition de la réponse immune à l'ARN était réalisée par différents inhibiteurs.

YOUMANS avait travaillé sur une seule souche bactérienne (Mycobacterium tuberculosis), dont le "parasitisme" est uniquement intracellulaire.

VENNEMAN et coll. pensent, dès 1972, que le véritable antigène pourrait être associé à l'ARN, dont le rôle serait celui d'un adjuvant. Ils vaccinent des souris avec de l'ARN ribosomal, extrait par le phénol à 65°C de ribosomes d'une souche de Salmonella typhimurium. Trente jours après cette vaccination, il s'avère que les animaux sont mieux protégés que par vaccin souche vivante (atténuée).

Il est surtout constaté que le niveau de protection est fonction de la quantité d'ARN injectée.

Par exemple : l'ARN ribosomal extrait de Streptococcus pneumoniae induit une protection de nature humorale et l'ARN ribosomal extrait de Klebsiella pneumoniae induit une protection de nature cellulaire.

[TRIEU-CUOT. P., G. GERBAUD, T. LAMBERT, and P. COURVALIN (1985) - In vivo transfer of genetic information between gram-positive and gram-negative bacteria, (EMBO J. 4:3583-3587)].

Ce mélange, injecté in vivo sur souris et cobayes, exerce une action sur les macrophages alvéolaires.

Cet effet "transitoire" se retrouve en dosant la phosphatase acide des plages d'hémolyse directe au contact des cellules spléniques de souris.

Le traitement par notre complexe thérapeutique et vaccinal est, quant à lui, suivi d'un effet immunostimulant cellulaire et humoral, avec une action spécifique et non spécifique, significative, sur les Helicobacter pylori. C'est l'organisme du patient lui-même qui est sollicité pour "rejeter les cellules infectées". On obtient une guérison par l'action des PMNs (Polymorphonuclear leukocytes) et des monocytes simultanément sollicités.
[ANDERSEN LP; NIELSEN H. Survival and ultrastructural changes of Helicobacter pylori after phagocytocis by human polymorphonuclear leukocytes and monocytes, APMIS; 1993 Jan: 101(1); 61-72]
[STEIGBIGEL, R. T., LAMBERT, L. H. & REMINGTON. J..S.: Phagocytic and bactericidal properties of normal human monocytes. J. Clim. Invest. 53: 131-142, 19741
[YAM, L.T., Li. C. Y. & CROSBY. W.H.: Cytochemical identification of monocytes and granulocytes, Am. J. Clin. Pathol. 55: 283-290, 1971].
Ce mécanisme thérapeutique permet donc de produire un clonage naturel grâce aux ARN
   (ribosomaux bactériens non spécifiques) opsonisés par l'adjuvant mis au point (combinaison de protéoglycanes membranaires. de collagène de type III, de chlorure de sodium).

Ce clonage induit une vaccination contre les idiotypes des anticorps, ainsi qu'une production d'anticorps contre le site de liaison des bactéries. Pour diminuer ou inhiber la réaction inflammatoire, il est nécessaire d'utiliser, lors des traitements par le complexe vaccinal, des corticoïdes (type Betaméthazone, par exemple) sous forme de phosphate disodique, à la dose de 20 à 60 mg, par voie I.V. ou I.M.

Cette action s'accompagne également d'une production d'interféron endogène, ainsi que d'une activation des cellules N.K.

Le but de notre complexe vaccinal immuno-modulateur est donc d'induire une réponse immunitaire locale et générale ayant pour effet d'empêcher ou au moins de réduire (jusqu'à un seuil d'auto-défense possible) la prolifération d'un agent infectieux introduit dans l'organisme.
- PRUUL, H., LEE, P. C., GOODWIN, C. S. & MACDONALD, P. J, - Interaction of Campylobacter pyloridis with human immune defence mechanisms, (J. Med. Microbiol. 23: 233-238, 1987).
- RATHBONE,B. J., WYATT. J. I., WORSLEY, B. W., SHIRES. S. E., TREJDOSIEWICZ. L. K., HEATLEY, R. V. & LOSOWSKY, M: S. - Systemic and local antibody response to gastric Campylobacter pyloridis in non-ulcer dyspepsia, (Gut 27: 642-647, 1986).
- STACEY, A, R., HAWTIN, P. R. & NEWELL, D. G. - Local immune responses to Helicobacter pylori ingections.In : Malgertheimer, P. & Ditschuneit, H. (Eds.) : Helicobacter pylori, Gastritis and Peptic Ulcer, (Springer Verlang, Berlin-Heidelberg, 1990, pp. 162-166).

Notre originalité thérapeutique consiste, entre autres, à modérer ou supprimer l'existence de "cellules suppressives" exerçant une action pro-infectieuse, à provoquer une réaction anti-ulcéreuse par réponse cellulaire et/ou humorale de défense. C'est la réponse thérapeutique au problème pressenti dès 1993 par Kist et Coll.

(KIST M; SPIEGELHALDER C; MORIKI T; SCHAEFER HE - Interaction of Helicobacter pylori (stain 151) and Campylobacter coli with human peripheral polymorphonuclear granulocytes(
et à prévenir les récidives infectieuses :
- BORODY T, ANDREWS P, MANCUSO N, JANKIEWICZ E, BRANDL S - Helicobacter pylori reinfection 4 years post-eradication; (Lancet 1992, 339:1295).
- BELL GD, POWELL KU, BURRIDGE SM, HARRISON G, RAMER B, WEIL J, et al - Reinfection or recudescence after apparently successful eradication of Helicobacter pylori infection : Implications for treatment of patients with duidenal ulcer disease, (Q J Med 1993, 86:375-382).

En conclusion, notre complexe thérapeutique agit par évolution dirigée produisant des molécules d'ARN, lesquelles, bloquent l'infection par l'Helicobacter pylori, et augmentent l'immunodéfense.

[SUERBAUM. S., C. JOSENHANS, and A. LABIGNE (1993) - Cloning and genetic characterization of the Helicobacter pyroli and Helicobacter mustelae flaB flagellin genes and construction of H. pylori flaA- and flaB-negative mutants by electroporation-mediated allelic exchange, (J. Bacteriol. 175:3278-3288).
- HAAS, R., T. F. MEYER, and J. P. VAN PUTTEN ( 1993) - Aflagellated mutants of Helicobacter pylori generated by genetic transformation fof naturally compétent strains using transposon shuttle mutagenesis, (Mol. Microbiol, 8:753-760)
- CHEN M, LEE A HAZELL S, HU P. LI Y - Protective immunisation against Helicobacter the need for stimulation of common mucosal immune system (abstract), (Gastroenterology 1993, 104 (suppl): A681)].

Il a par ailleurs été constaté lors des différents essais cliniques auxquels il a été procédé, que le complexe de l'invention pouvait suppléer avec succès aux traitements conventionnels. par triple thérapie notamment, dans les cas de résistances bactériennes notoires.

### Techniques d'administration du complexe vaccinal

Le complexe vaccinal peut être administré par voie orale, ou par voie parentérale :
* soit par injection intraveineuse directe
* ou par perfusion lente
* ou par injection sous-cutanée.
* ou par voie transdermique (par 24 h.)

Ces diverses techniques ont été expérimentées avec succès.

Les dosages journaliers et leur fréquence dépendent beaucoup de l'état du patient. Un surdosage ne présente aucun risque, compte tenu de la non toxicité du complexe.

Par voie intraveineuse, on peut utiliser des séquences d'une semaine par mois, chaque jour de la semaine de traitement comportant une perfusion lente de 500 ml d'une solution renfermant :
- 0.9 % de chlorure de sodium
- 40 µg de fractions saccharidiques membranaires (protéoglycanes de Klebsiella pneumoniae)
- 30 µg d'ARN (ribosomal) de :
   * Helicobacter pylori 7µg
   * Diplococcus pneumoniae 7µg
   * Streptococcus pyogènes (A 12) 7µg
   * Klebsiella pneumoniae 7 µg
   * Hemophilus influenzae 2 µg
- 10 µg de collagène type III décrit ci-dessus
- 8 mg de phosphate disodique de Bétaméthazone (soit 2 ml de soluté injectable)

A ce traitement par perfusion I.V. lente. peut succéder un traitement par injections sous-cutanées sur les patients pouvant être suivis de façon ambulatoire, chaque injection contenant :
- 40 µg de fractions saccharidiques membranaires (protéoglycanes de Klebsiella pneumoniae)
- 30 µg d'ARN (ribosomal) de :
   * Helicobacter pylori 7µg
   * Diplococcus pneumoniae 7µg
   * Streptococcus pyogène (A 12)7µg
   * Klebsiella pneumoniae 7 µg
   * Hemophilus influenzae 2 µg
- 10 µg de collagène type III décrit ci-dessus
- 0.5 ml de chlorure de sodium à 0,9 %
- 4 mg de phosphate disodique de Bétaméthazone (soit 1 ml de soluté injectable).
   Ce traitement peut être poursuivi quelques semaines.

Par voie orale :
- par comprimés.
   2 comprimés par jour, en une seule prise le matin à jeun, chaque comprimé renfermant : - 400 µg de fractions sacchraridiques membranaires (protéoglycanes de Klebsiella pneumoniae)
   - 300 µg d'ARN (Ribosomal) de :
      * Helicobacter pyroli 70 µg
      * Diplococcus pneumoniae 70 µg
      * Streptococcus pyogènes (A12) 70 µg
      * Klebsiella pneumoniae 70 µg
      * Hemophilus influenzae 20 µg
   - 100 mg de Collagène type III décrit ci-dessus
   - 2 mg de phosphate disodique de Betamethazone

Ce traitement peut être octroyé à raison de 2 comprimés par jour pendant un mois, suivi de périodes de rappel de deux comprimés par jour, une semaine par mois pendant 3 mois.

### Par voie transdermique

Système thérapeutique transdermique adhésif composé d'un réservoir et d'une membrane perméable assurant le passage continu des principes actifs à travers la peau et dans la circulation sanguine à vitesse constante.

Le dispositif doit être collé sur une surface cutanée saine, sèche et peu pileuse (paroi latérale de l'abdomen ou du thorax par exemple).

Il comporte :
- Polymère adhésif
- Support de l'adhésif : polyéthylène
- Filtre protecteur polyester siliconé

Son contenu est le contenu d'un comprimé, et sa posologie est identique à la voie orale (à raison d'un "patch" pour 2 comprimés quotidiens).

Les exemples suivants, non limitatifs, sont communiqués pour illustrer les résultats concrets de notre complexe vaccinal thérapeutique.

### Exemple 1

M. Robert G., 64 ans, est hospitalisé à la suite d'épigastralgies, de pyrosis et de douleurs abdominales associées à un trouble du transit avec alternance diarrhée - constipation. L'endoscopie digestive retrouve une pathologie de reflux gastro-oesophagien par béance du cardia entraînant une oesophagite et un ulcère peptique du bas oesophage.

Des biopsies sont pratiquées de même qu'un test rapide à l'uréase, Celui-ci, de même que l'anatomopathologie et la culture, confirment la présence d'Helicobacter pylori.

Le traitement conventionnel (antisécrétoire et deux antibiotiques) est prescrit. La trithérapie n'entraîne pas de guérison clinique.

Six semaines après la fin du traitement, le contrôle d'éradication par test respiratoire à l'urée marquée au ¹³C fait conclure en la prolifération des bactéries par sa positivité.

Le traitement par le complexe vaccinal objet de l'invention est alors pratiqué sous forme d'injections sous-cutanées.

Un mois après, on constate la guérison clinique et le test respiratoire à l'urée marquée au carbone-13 est négatif.

Six mois après, un nouveau contrôle par test respiratoire à l'urée marquée ¹³C et l'endoscopie de contrôle montrent une guérison acquise.

Depuis un an, la guérison est définitive.

### Exemple 2

M. Serge Y., 48 ans, présente une gastrite antrale de type B. Traitement par complexe immunomodulateur (les seuls traitements antérieurs étaient des pansements gastriques) sous forme IV. La guérison clinique est acquise quinze jours après la séquence thérapeutique. Les contrôles (test respiratoire à l'urée marquée au ¹³C) sont négatifs depuis un an.

### Exemple 3

M. Pierre K., présente un ulcéré duodenal confirmé à l'endoscopie (+Biopsie, test à l'uréase, tests ELISA).

Le traitement par voie orale est alors instauré. Trois semaines après, la guérison clinique est obtenue.

Six semaines après. le contrôle par test respiratoire à l'urée marquée ¹³C confirme l'éradication.

Six mois après. aucune récidive n'est enregistrée, et le test Elisa montre un taux d'anticorps non significatif (< 50 %).

### Exemple 4

Mme Sarah L. présente un ulcère duodénal associé à une gastrite de type B.

On relève la présence de cancer gastrique dans sa fratrie. Un bilan complet pratiqué montre la positivité de tous les tests par méthode invasive : culture, histologie, amplification du génome viral (PCR), test à l'uréase.

Le traitement par voie intraveineuse sur une semaine puis par rappels sous-cutanés sur six mois est alors instauré.

Etant donné le haut risque familial, une endoscopie avec biopsie est pratiquée dès le troisième mois : PCR cytologie, culture, CLO test, sont négatives.

Au sixième mois, un test respiratoire (¹³C) confirme la guérison clinique.

## Revendications

1. Complexe immunomodulateur thérapeutique spécifique, ***caractérisé en ce qu***'il comprend :
- des molécules duales constituées par le couplage d'un bras fonctionnel d'acides aminés assurant la liaison à une cible, avec un bras génétique d'ARN correspondant à la description codée de la composition du bras fonctionnel.
- des fractions membranaires bactériennes, glycopeprides et/ou lipopolysaccharides,
les acides ribonucléiques (ARN) étant d'origine ribosomale et extraits des souches choisies dans le groupe suivant : Helicobacter pylori, hepaticus, coronari, Campylobacter ou d'un mélange de celles-ci.

2. Complexe immunomodulateur selon la Revendication 1, ***caractérisé en ce que*** les acides aminés sont des acides aminés de collagéne.

3. Complexe immunomodulateur selon la Revendication 2, ***caractérisé en ce que*** les acides aminés de collagéne sont choisis dans le groupe suivant : acide aspartique. hydroxyproline, thréonine, sérine, acide glutamique, proline, glycine, alanine, valine, méthionine, isoleucine, leucine, tyrosine, phénylaline, lysine, histidine, arginine, ou d'un mélange de ceux-ci.

4. Complexe immunomodulateur conforme à l'une des Revendications 1 à 3 pour son utilisation dans le traitement des affections par bactéries Helicobacter, par la production d'anticorps et la production d'interféron endogène.

5. Complexe immunomodulateur conforme à l'une des Revendications 1 à 3 pour son utilisation comme vaccin anti-idiotypique contre les idiotypes des anticorps anti-bactériens permettant d'éviter notamment les récidives de la pathologie initiale du tractus digestif.

6. Complexe immunomodulateur conforme à l'une des Revendications 1 à 3 pour son utilisation contre les résistances bactériennes aux traitements conventionnels par antibiotiques ou autres.

7. Complexe immunomodulateur et vaccinal spécifique anti-Helicobacter selon l'une des Revendications précédentes, ***caractérisé en ce qu***'il est présenté sous un conditionnement permettant l'administration simultanée d'anti-inflammatoires majeurs du type corticoïdes, d'antibiotiques, d'antisécrétoires, (inhibiteurs de la pompe à protons, type Oméprazole ou anti H2...) ou autres produits à effets bactériostatiques, bactéricides ou bactériolytiques, pour éradiquer Helicobacter générant une pathogénèse par facteurs liés à la bactérie (production de différentes cytotoxines, de médiateurs de l'inflammation : Interleukine I, facteur alpha de nécrose tumorale (Tumor necrosis factor alpha)), ou par facteurs liés à l'hôte.

8. Complexe immunomodulateur selon la Revendication précédente, ***caractérisé par*** un conditionnement sous une forme telle qu'il peut être administré par différentes voies : perfusions, injections intraveineuses, injections sous-cutanées, dispositifs transdermiques, ou per os.

## Patentansprüche

1. Spezifischer, therapeutischer Immunmodulatorkomplex, **gekennzeichnet dadurch, dass** er umfasst:
- Doppelmoleküle, welche aus der Kopplung eines funktionellen Arms aus Aminosäuren, welcher die Bindung an einem Target sicherstellt, mit einem genetischen RNS-Arm bestehen, welcher der kodierten Beschreibung der Zusammensetzung des funktionellen Arms entspricht,
- bakterielle Membranfraktionen, Glycopeptide und/oder Lipopolysaccharide,
wobei die Ribonukleinsäuren (RNS) ribosomaler Herkunft sind und aus Stämmen extrahiert werden, die aus der folgenden Gruppe ausgewählt werden: Helicobacter pylori, Helicobacter hepaticus, Helicobacter coronari, Campylobacter, oder einer Mischung aus ihnen.

2. Immunmodulatorkomplex nach Anspruch 1, **dadurch gekennzeichnet, dass** die Aminosäuren aus Aminosäuren von Collagen bestehen.

3. Immunmodulatorkomplex nach Anspruch 2, **dadurch gekennzeichnet, dass** die Aminosäuren von Collagen aus der folgenden Gruppe ausgewählt werden: Asparaginsäure, Hydroxyprolin, Threonin, Serin, Glutaminsäure, Prolin, Aminoessigsäure, Aminopropionsäure, Valin, Methionin, Isoleucin, Leucin, Tyrosin, Phenylalalin, Lysin, Histidin, Arginin, oder einer Mischung aus ihnen.

4. Immunmodulatorkomplex nach einem der Ansprüche 1 bis 3, zu seiner Verwendung bei der Behandlung von Erkrankungen durch Helicobacter-Bakterien, durch die Produktion von Antikörpern und die Produktion von endogenem Interferon.

5. Immunmodulatorkomplex nach einem der Ansprüche 1 bis 3, zu seiner Verwendung als anti-idiotypischer Impfstoff gegen die Idiotypen von antibakteriellen Antikörpern, wodurch insbesondere die Vermeidung der Rückfälle in die ursprüngliche Pathologie des Verdauungstraktes ermöglicht wird.

6. Immunmodulatorkomplex nach einem der Ansprüche 1 bis 3, zu seiner Verwendung gegen die bakteriellen Resistenzen bei herkömmlichen Behandlungen mit Antibiotika oder dergleichen.

7. Immunmodulatorkomplex und spezifischer Anti-Helicobacter Impfkomplex nach einem der vorhergehenden Ansprüche, **dadurch gekennzeichnet, dass** er sich in einer Handelsform präsentiert, welche die gleichzeitige Verabreichung von Hauptgruppen entzündungshemmender Mittel vom Typ Corticoide, Antibiotika, Antisekretionsmittel, (Hemmstoffe der Protonenpumpe, des Typs Omeprazol oder Anti-H2... ) oder anderer Produkte mit Wirkungen gestattet, die das Bakterienwachstum hemmen, Bakterien töten oder bakteriolytische Wirkung haben, um den Helicobacter vollständig zu vernichten, der eine Krankheitserregung durch Faktoren erzeugt, die mit der Bakterie im Zusammenhang stehen (Produktion von unterschiedlichen Zellengiftstoffen, von Entzündungsbeschleunigern: Interleukin I, Tumor-Nekrose-Faktor-alpha (Tumor necrosis factor alpha)), oder durch Faktoren, die mit dem Wirt im Zusammenhang stehen.

8. Immunmodulatorkomplex nach dem vorhergehenden Anspruch, **gekennzeichnet durch** eine Handelsform in einer Gestalt, welche die Verabreichung auf verschiedenen Wegen ermöglicht: Tropfinfusionen, intravenöse Injektionen, subkutane Injektionen, transdermatologische Vorrichtungen (Hautübertragung), oder orale Verabreichung.

## Claims

1. Specific therapeutic immunomodulatory complex, ***characterized in that it*** comprises:
- dual molecules constituted by the coupling of a functional amino acid arm, ensuring binding to a target, with a genetic RNA arm corresponding to the coded description of the composition of the functional arm,
- bacterial membrane fractions glycopeptides and/or lipopolysaccharides,
the ribonucleic acids (RNA) being of ribosomal origin and extracted from strains chosen from the following group: Helicobacter pylori, hepaticus, coronari, Campylobacter or from a mixture thereof.

2. Immunomodulatory complex according to Claim 1, ***characterized in that*** the amino acids are amino acids from collagen.

3. Immunomodulatory complex according to Claim 2, ***characterized in that*** the amino acids from collagen are chosen from the following group: aspartic acid, hydroxyproline, threonine, serine, glutamic acid, proline, glycine, alanine, valine, methionine, isoleucine, leucine, tyrosine, phenylaline, lysine, histidine, arginine, or a mixture thereof.

4. Immunomodulatory complex according to one of Claims 1 to 3, for its use in the treatment of diseases caused by Helicobacter bacteria, by the production of antibodies and the production of endogenous interferon.

5. Immunomodulatory complex according to one of Claims 1 to 3, for its use as an anti-idiotype vaccine against the idiotypes of anti-bacterial antibodies which make it possible to avoid, in particular, recidivations of the initial digestive tract pathology.

6. Immunomodulatory complex according to one of Claims 1 to 3, for its use against bacterial resistance to conventional antibiotic treatments and the like.

7. Anti-Helicobacter-specific immunomodulatory and vaccine complex according to one of the preceding claims, ***characterized in that it*** is presented in a packaging allowing the simultaneous administration of major antiinflammatory agents of the corticoid type, of antibiotics, of antisecretory agents, (proton pump inhibitors, of the type including Omeprazole or anti-H2, and the like) or other products with bacteriostatic, bactericidal or bacteriolytic effects, for eradicating Helicobacter generating pathogeneses by factors linked to the bacterium (production of various cytotoxins, of inflammation mediators: Interleukin I, tumour necrosis factor alpha), or by factors linked to the host.

8. Immunomodulatory complex according to the preceding claim, ***characterized by*** a packaging in the form such that it can be administered by various routes: perfusions, intravenous injections, subcutaneous injections, transdermal devices, or per os.
